Europäisches Patentamt

European Patent Office

Office européen des brevets

(19)

(11) Veröffentlichungsnummer: **0 450 489 A1**

## (12) EUROPÄISCHE PATENTANMELDUNG

(21) Anmeldenummer: **91104872.6**

(22) Anmeldetag: **27.03.91**

(51) Int. Cl.⁵: **A61F 13/20**

(30) Priorität: **03.04.90 DE 4010701**

(43) Veröffentlichungstag der Anmeldung:
**09.10.91 Patentblatt 91/41**

(84) Benannte Vertragsstaaten:
**AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Theis, Dirk, Dr.**
**Höchster Schlossplatz 4**
**W-6230 Frankfurt am Main 80(DE)**
Erfinder: **Fester, Walter, Dr.**
**Peter-Konrad-Strasse 24**
**W-8424 Saal(DE)**

(54) **Tampon und Verfahren zu dessen Herstellung.**

(57) Beschrieben wird ein Tampon aus Endlosfilamenten auf Basis eines Chemiefaserkabels mit einem Wasserhaltevermögen von mindestens 1000 Gew.-%, der im wesentlichen aus parallel zur Längsachse angeordneten und durchgehenden Filamenten besteht und praktisch nur auf den beiden begrenzenden Querschnittsflächen freie Filamentenden aufweist.

EP 0 450 489 A1

Die vorliegende Erfindung betrifft ein Tampon aus endlosen Filamenten und ein Verfahren zu dessen Herstellung. Tampons werden üblicherweise aus Vliesen, insbesondere aus Kardenvliesen, hergestellt. Diese bestehen im allgemeinen aus Cellulosefasern.

Aus der US-A-4,627,849 sind Tampons bekannt, deren kennzeichnendes Merkmal im Aufbringen einer sogenannten "Mikrowelligkeit" besteht. Darunter ist eine Anordnung der gerkräuselten Einzelfilamente zu verstehen, bei der die Knickpunkte mehrerer Filamente so zusammengefaßt sind, daß sich insgesamt eine Welligkeit der eingesetzten Fasermatten ergibt. Aus den Fig. 7 bis 9 dieses Patents ist auch eine Herstellungweise bekannt, bei der eine Fasermatte ausgehend von der Mitte aufgerollt wird und anschließend zum Endprodukt verpresst wird. Diese Ausführungsform weist an der Oberfläche eine Vielzahl freier Filamentenden auf. Solche freien Filamentenden können zu Hautreizungen und allergischen Reaktionen führen und sind daher auf der Oberfläche des Tampons unerwünscht. In diesem Patent wird auch die Verwendung endloser Faserkabel (in folgenden Kabel genannt) vorgeschlagen, doch wird diese Möglichkeit dort nicht näher ausgeführt.

Es wurde jetzt gefunden, daß man Tampons ausgehend von Kabeln in einfacher Weise herstellen kann. Es entfallen mit diesem Verfahren die Schritte der textilen Vorbehandlung die durch die Verwendung von Kardenvliesen nötig waren. Ferner erhält man ein Produkt, das einen erheblich reduzierten Gehalt an freien Filamentenden entlang der Oberfläche des Tampons aufweist.

Die vorliegende Erfindung betrifft einen Tampon gemäß der in Anspruch 1 gegebenen Definition.

Das erfindungsgemäß eingesetzte Fasermaterial muß ein Wasserhaltevermögen (nach DIN 61640) von mindestens 1000 Gew.-% besitzen, d.h. es muß mindestens eine Wassermenge halten können, die mindestens dem Zehnfachen seines Eigengewichts entspricht. Üblicherweise beträgt das Wasserhaltevermögen 1000-2000 Gew.-%, vorzugsweise jedoch mindestens 1500 Gew.-%.

Bei dem Fasermaterial kann es sich um Cellulose handeln, vorzugsweise um nicht modifizierte Cellulose. Beispiele dafür sind nach dem Direktlösungsverfahren ersponnene Cellulose, z.B. aus N-Methylmorpholin-N-oxid als Lösungsmittel ersponnene Cellulose, oder Regeneratcellulose, wie Kupferseide oder insbesondere Viskose.

Das eingesetzte Kabel besitzt eine Stärke von etwa 30 bis 180 ktex, insbesondere von etwa 70 bis 120 ktex. Die Auswahl der Kabelstärke hängt im wesentlichen von den gewünschten Dimensionen des Endproduktes ab.

Der Einzelfadentiter des zu verwendenden Kabels liegt üblicherweise zwischen etwa 1 und 10 dtex, insbesondere zwischen etwa 2 bis 5 dtex. Die Auswahl dieser Größe wird nach oben durch die nötige mechanische Festigkeit des Tampons vorgegeben, während die untere Grenze hauptsächlich durch die Wirtschaftlichkeit des Faserherstellungsverfahrens bestimmt wird.

Das einzusetzende Kabel muß eine Kräuselung aufweisen, um dem Endprodukt eine ausreichende Festigkeit zu verleihen. Es können alle an sich üblichen Arten der Kräuselung angewendet werden, wie Zahnradkräuselung und insbesondere Stauchkammerkräuselung. Insbesondere bei Viskosefilamenten kann die durch den Viskoseprozeß bedingte Kräuselung ohne weitere mechanische Behandlung ausreichen.

Wesentliche Merkmale des erfindungsgemäßen Tampons sind der Aufbau aus Endlosfilamenten und der im wesentlichen parallele Verlauf dieser Filamente zur Längsaches des Tampons. Dadurch wird es möglich, daß freie Filamentenden praktisch nur an den begrenzenden Querschnittsflächen des Zylinders auftreten und daß die später mit der Haut in Kontakt tretende Zylinderfläche praktisch keine solchen freien Filamentenden aufweist.

Der erfindungsgemäße Tampon kann direkt aus dem Kabelmaterial hergestellt werden oder er kann zusätzlich mit einem Hüllvlies versehen werden.

Solche Hüllvliese sind an sich bekannt. Dabei kann es sich beispielsweise um Ultraleichtvliese auf Basis von Polyester/Polyethylen Bikomponentenfasern handeln. Das Flächengewicht solcher Hüllvliese beträgt vorzugsweise weniger oder gleich 15 g/m$^2$, besonders bevorzugt etwa 10 g/m$^2$.

Der erfindungsgemäße Tampon kann mit einem Rückholfaden versehen werden. Dieser Faden kann entweder während des kontinuierlichen Herstellungsprozesses eingebracht werden oder erst nach dem Zurechtschneiden des geformten Kabelstranges auf die gewünschte Größe.

Bei dem Rückholfaden kann es sich um Zwirne oder Garne aus natürlichen und/oder synthetischen Fasern handeln. Insbesondere verwendet man Zwirne oder Garne aus einem thermoplastischen Polymeren, das nach dem Einbringen in den Tampon mit den es umgebenen Filamenten verklebt werden kann, beispielsweise durch Erhitzen über den Erweichungspunkt des thermoplastischen Polymeren. Der Rückholfaden wird vorzugsweise in der Längsachse des Tampons angebracht. Der erfindungsgemäße Tampon kann in Analogie zu einem Verfahren hergestellt werden, daß auch bei der Herstellung von Zigarettenfiltern verwendet wird. Die Funktionsweise und daraus resultierend die Ausgestaltungen von Tampons und solchen Filtern sind nicht miteinander vergleichbar. Tampons müssen so ausgestaltet sein, daß ihre Oberfläche eine möglichst große und einheitliche Flüssigkeitsaufnahme bewirkt, während die Oberfläche von Zigarettenfiltern möglichst gasdicht abgeschlossen sein muß, um die Funktion eines solchen Filters zu gewährleisten. Es lag also nicht nahe, ein solches Verfahren zur Herstellung von Tampons zu verwenden.

Das erfindungsgemäße Verfahren ist gekennzeichnet durch die in Anspruch 7 definierten Maßnahmen.

Zum Auffächern des Kabels ist jedes an sich bekannte Verfahren geeignet. Im allgemeinen wird ein einlaufendes Kabel mechanisch und/oder durch Fluidstrahlen geöffnet. Beispiele für solche Verfahren findet man in der US-A-3,156,016 und in der DE-A-1,435,417.

Das Auffächern kann gegenbenenfalls durch elektrostatische Aufladung unterstützt werden.

Das Auffächern dient unter anderem einer Vergleichmässigung des Verlaufes der Einzelfilamente.

Nach diesem Schritt wird das aufgefächerte Kabel in ein Führungselement eingebracht, wodurch eine Kompaktierung unter Erhalt des im wesentlichen parallelen Verlaufs der Einzelfilamente erfolgt. Bei dem Führungselement handelt es sich üblicherweise um eine trichterförmige Vorrichtung, in deren weite Öffnung das aufgefächerte Kabel eingeleitet wird und in deren verengtem Teil das Kabel auf die gewünschte Stärke verdichtet wird.

Vorzugsweise wird das aufgefächerte Kabel mit einer höheren Geschwindigkeit in die trichterförmige Vorrichtung eingeführt als daraus abtransportiert. Die Geschwindigkeitsdifferenz wird in Kompressionsenergie umgewandelt und dient zur Verdichtung des Kabels im verengten Teil der Vorrichtung.

Während des Verdichtungsvorganges kann ein Rückholfaden eingebracht werden, vorzugsweise durch kontinuierliches Zuführen eines thermoplastischen Fadens in das aufgefächerte Kabel, so daß dieses Faden beim Verdichten am Ort der Längsachse des Tampons verläuft.

Das kompaktierte Kabel wird anschliessend verpresst, indem man senkrecht zur Zylinderoberfläche einen Druck aufbringt. Eine geeignete Vorrichtung dafür ist z.B. in den Fig. 4, 4a und 4b der US-A-4,627,849 beschrieben.

Anschließend oder vor dem Verpressen wird das kompaktierte Kabel in die gewünschte Länge geschnitten. Danach wird ein gegebenenfalls eingebrachter Rückholfaden teilweise und gegebenenfalls unter Verstreckung aus dem Tampon herausgezogen und anschließend mit dem Fasermaterial verklebt. Vorzugsweise erfolgt dies durch Erwärmen eines thermoplastischen Materials über dessen Erweichungspunkt.

Eine weitere Ausgestaltung des erfindungsgemäßen Verfahrens umfaßt das Einhüllen des kompaktierten Kabels in ein Hüllvlies. Dazu kann eine an sich übliche Vorrichtung verwendet werden, beispielsweise wie in Fig. 3 der US-A-4,627,849 gezeigt. Das Einhüllen kann aber auch diskontinuierlich erfolgen, beispielsweise nach dem Zuschneiden der kompaktierten Kabel. Vorzugsweise erfolgt das Einhüllen kontinuierlich, wie in Anspruch 9 definiert.

Die erfindungsgemäßen Tampons sind vielseitig einsetzbar, beispielsweise für medizinische Zwecke oder insbesondere für die Frauenhygiene. Sie zeichnen sich durch eine hohe Saugkraft und durch eine besonders gute Verträglichkeit aus. Insbesondere sind sie auf einfache und wirtschaftliche Weise herzustellen.

**Patentansprüche**

1. Tampon aus Endlosfilamenten bestehend im wesentlichen aus einem Kabel aus gekräuselten Chemiefasern, welches ein Wasserhaltevermögen von mindestens 1000 Gew.-% besitzt, wobei das Kabel eine Stärke von etwa 30-180 ktex und die Filamente in diesem Kabel einen Einzelfadentiter von etwa 1 bis 10 dtex aufweisen, und der Tampon im wesentlichen aus parallel zur Längsachse angeordneten und durchgehenden Filamenten besteht und praktisch nur auf beiden begrenzenden Querschnittsflächen freie Filamentenden aufweist.

2. Tampon gemaß Anspruch 1, dadurch gekennzeichnet, daß es sich bei den Chemiefasern um Cellulosefasern handelt.

3. Tampon gemäß Anspruch 2, dadurch gekennzeichnet, daß es sich bei den Cellulosefasern um Viskosefasern hanelt.

4. Tampon gemäß einem der Ansprüche 1 bis 3, dadurch gekennzeichnet, daß der Einzelfadentiter etwa 2 bis 5 dtex beträgt.

5. Tampon gemäß einem der Ansprüche 1 bis 4, dadurch gekennzeichnet, daß dieser aus einem Kern aus im wesentlichen parallel zur Längsachse angeordneten und durchgehenden Filamenten besteht, welcher mit einem Hüllvlies versehen ist.

6. Tampon gemäß einem der Ansprüche 1 bis 5, dadurch gekennzeichnet, daß dieser mit einem Rückholfaden aus thermoplastischem Material versehen ist, der in der Längsachse des Tampons in diesen eingebracht ist und mit den ihn umgehenden Filamenten verklebt ist.

7. Verfahren zur Herstellung eines Tampons gemäß Anspruch 1 ausgehend von einem Kabel aus gekräuselten Chemiefasern, welches ein Wasserhaltevermögen von mindestens 1000 Gew.-% besitzt, das eine Kabelstärke von etwa 30 - 180 ktex besitzt und dessen Filamente einen Einzelfadentiter von etwa 1-10 dtex aufweisen, umfassend die Schritte:

i) Auffächern des Kabels in einer dafür geeigneten Vorrichtung, um den Verlauf der Einzelfilamente zu vergleichsmäßigen,

ii) Einbringen des aufgefächerten Kabels in ein Führungselement, welches eine gegenüber dem aufgefächeren Kabel stark verringerte Querschnittsfläche aufweist, um das Kabel unter Erhalt des im wesentlichen parallelen Verlaufs der Einzelfilamente zu kompaktieren,

iii) Schneiden des kompaktierten Kabels in zylinderförmige Stücke mit einer dem gewünschten Anwendungszweck angepassten Länge, und

iV) Verpressen, um den Tampon die gewünschte mechanische Festigkeit und die gewünschte Stärke zu verleihen, wobei die Schritte iii) und iv) auch in umgekehrter Reihenfolge ausgeführt werden können.

8. Verfahren zur Herstellung eines Tampons gemäß Anspruch 7, dadurch gekennzeichnet, daß man vor Schritt ii) einen thermoplastischen Faden in das aufgefächerte Kabel so einbringt, daß dieser in Schritt iii) in der Längsachse des kompaktierten Zylinders verläuft.

9. Verfahren zur Herstellung eines Tampons gemäß einem der Ansprüche 7 bis 8, dadurch gekennzeichnet, daß man den nach Schritt ii) kompaktierten Zylinder mit.einem Hüllvlies versieht, indem man eine Bahn dieses Hüllvlieses dem kompaktierten Zylinder kontinuierlich zuführt und dieses mit einer dafür geeigneten Vorrichtung um besagten Zylinder legt.

10. Verfahren zur Herstellung eines Tampons, gemäß einem der Ansprüche 7 bis 10, dadurch gekennzeichnet, daß man in Schritt ii) ein trichterförmiges Führungselement wählt, und daß man das aufgefächerte Kabel mit einer höheren Geschwindigkeit in den Trichter einbringt als dieses aus der Verengung des Trichter abgezogen wird.

Europäisches
Patentamt

**EUROPÄISCHER RECHERCHENBERICHT**

Nummer der Anmeldung

EP 91104872.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl⁵) |
|---|---|---|---|
| A | <u>DE - C - 551 712</u><br>(ACKERMANN)<br>* Gesamt * | 1 | A 61 F 13/20 |
| A | <u>US - A - 4 335 721</u><br>(MATTHEWS)<br>* Gesamt * | 1 | |
| A | <u>US - A - 4 787 895</u><br>(STOKES)<br>* Gesamt * | 1 | |
| A | <u>DE - B - 1 491 169</u><br>(JOHNSON & JOHNSON)<br>* Fig. 5 * | 1,7 | |
| A | <u>GB - A - 2 200 285</u><br>(KIMBERLY-CLARK)<br>* Fig. 1,8 * | 1,7 | |
| A | <u>DE - A - 2 802 101</u><br>(DR. HAHN)<br>* Fig. 2,3 * | 1,7 | RECHERCHIERTE SACHGEBIETE (Int Cl⁵)<br><br>A 61 F 13/00 |

Der vorliegende Recherchenbericht wurde für alle Patentansprüche erstellt.

| Recherchenort | Abschlußdatum der Recherche | Prüfer |
|---|---|---|
| WIEN | 11-06-1991 | KNAUER |